# EUROPEAN PATENT APPLICATION

(11) **EP 4 035 589 A1**
(43) Date of publication of application: **03.08.2022**
(21) Application number: 21154829.2
(22) Date of filing: 02.02.2021
(51) Int. Cl.: A61B 5/00

(54) **DETECTION AND METROLOGY OF ALLERGEN SKIN REACTION**

(71) Applicant: Hippocreates, 3460 Bekkevoort (BE)
(72) Inventor: Hofkens, Wim, 2460 Kasterlee (BE); De Roovere, Peter, 9000 Gent (BE)
(74) Representative: IP HILLS NV

(57) **Abstract**

A system (1) for detection and metrology of an allergen skin reaction of an individual, comprising:
- hosting a body part (10) of said individual and subjecting a skin surface area (11) of said body part (10) to an allergen skin reaction test;
- successively illuminating said skin surface area (11) having been subjected to said allergen skin reaction test under one or more predetermined illumination angles (20);
- imaging, from a fixed position and for each of said predetermined illumination angles (20), said illuminated skin surface (21); and
- determining one or more illumination-angle-dependent variations (202) in light intensity (201) imaged by said acquisition means (103), wherein said illumination-angle-dependent variations (202) are caused by one or more wheals (2) on said skin surface area (11).

## Description

### Technical Field

The present disclosure generally relates, amongst others, to allergy detection and more specifically to methods, devices, and systems for detecting allergies. More particularly, it relates to automating detection of allergic disease through skin reaction.

### Background

An allergy, also known as an allergic disease, corresponds to a condition caused by hypersensitivity of the immune system of an individual to one or more substances, also referred to as allergens, present in the environment of this individual. In other words, an allergic response, medically known as an immediate type I hypersensitivity allergic response, is triggered by an apparent excessive response of the immune system of the human body to harmless environmental antigens, referred to as allergens. More particularly, an allergy is a reaction of the human body when for example an allergen comes in contact with the human body. Several different types of allergy exist such as for example hay fever, food allergies, atopic dermatitis, allergic asthma, anaphylaxis, allergies to medicines, etc. Before a clinically significant allergic response can occur, an individual must be sensitized to an allergen. During sensitization, individuals who develop allergies default to a strong Th2 CD4 T-cell response when exposed to the allergen. These Th2 cells are principally characterized by the production of the cytokines IL-4, IL-5, and IL-13. In excessive response, IL-4 and IL-13 will instruct the allergen-specific B-cells to class switch to immunoglobulin or IgE. The IgE antibodies with specificity for the offending allergen will coat the surface of mast cells located in connective and subepithelial tissues throughout the body. Mast cells bind the IgE via their cell surface high-affinity IgE receptor also known as FcεRI, resulting in the antigen-binding regions of the antibody oriented away from the cell surface. The mast cells are now said to be sensitized against the offending allergen.

Re-exposure to the sensitizing allergen will cause cross-linking of the IgE on the mast cell surface by the allergen, which will stimulate the mast cell's activation and release of the chemical mediators of the allergic response. The chemical mediators released from the mast cell include histamine. This organic nitrogenous compound is the main actor in the local inflammatory reaction, its biological effects include dilation of blood vessels, increased vascular permeability, and transient contraction of smooth muscles. These cascades together with the mast cell activation that stimulates the rapid synthesis and secretion of eicosanoids, comprising for example prostaglandins and leukotrienes, both of which are derived from arachidonic acid, will cause the typical allergic reaction.

Symptoms or clinical manifestation of these allergies may comprise one or more of for example red eyes, an itchy rash, sneezing, shortness of breath, swelling, rhinitis, asthma, urticaria, etc. The clinical manifestations of allergic responses vary with the anatomical site of the allergic reaction. For example, allergic rhinitis develops in response to inhaled allergens such as pollen that stimulate mast cells in the nasal mucosa, resulting in increased mucus secretion. On the other hand, allergic asthma is caused by the activation of bronchial mast cells and is characterized by airway obstruction caused by mucus secretion, inflammation, and bronchial smooth muscle contraction. Some symptoms may result in mild discomfort for the individual, while other symptoms or a combination thereof may result in severe health risks endangering the individual's life.

The most severe form of allergy is anaphylaxis, caused by the systemic activation of sensitized mast cells. This may occur in response to bee stings or ingested for example nuts or shellfish, the allergens from which get absorbed into the circulation. The systemic reaction causes oedema in many tissues, accompanied by a fall in blood pressure and bronchoconstriction, creating a potentially life-threatening situation.

An allergen is a type of antigen that produces an abnormally vigorous immune response in which the immune system of an individual fights off a perceived threat that would otherwise be harmless to the body. Sensitivities to allergens vary widely from one person or from one animal to another. Additionally, a very broad range of substances can be allergens to sensitive individuals. An allergen may be for example a suspended substance in the environment and which may be breathed in by an individual, such as for example pollen. An allergen may be for example ingested by an individual, such as for example food or medicines. An allergen may for example come in contact with the skin of the individual and provoke an immune response on the skin of the individual, such as for example dust mites, fabrics, metals, insect stings, insect venom, animal dander, or allergen gaseous or liquid or solid substances.

Treatments for allergies include the avoidance of known allergens. Other treatments for allergies involve the use of one or more medications such as for example steroids and/or antihistamines and/or adrenaline in severe reactions. Allergen immunotherapy, which gradually exposes people to larger and larger amounts of allergen, is for example useful for some types of allergies such as hay fever and reactions to insect bites.

However, it is not always possible to completely prevent the exposure of an allergic individual to an allergen. For example, an allergy to a medicine is usually only identified when the medicine is ingested or injected into the body and provokes an allergic reaction. This remaining existing risk for encounter between the individual and the allergen to which he or she is allergic could potentially threaten the individual's life.

It is therefore important to clearly identify allergens to which an individual is allergic. This way, an effective personalized treatment may be properly administrated to or foreseen for the individual. A common way to confirm allergies is to use the underlying hyper-sensitivity as a mechanism. The most utilized skin test evaluating IgE-driven allergic disorders is through the epicutaneous, also referred to as the prick/puncture route. Skin allergy testing comprises a range of methods for medical diagnosis of allergies that attempts to provoke a small, controlled, allergic response. Typical methods of allergen skin reaction testing comprise introducing a microscopic amount of an allergen to a patient's skin by various means, for example on the forearm or on the back of the patient. The most used allergen skin reaction tests are skin prick tests: the skin of a patient is pricked or punctured with a needle or a pin containing a small amount of the allergen, thereby making a micro-incision on the skin of the patient and simultaneously bringing the allergen in contact with the body. Alternatively, making a micro-incision on the skin of a patient succeeds the deposition of a microscopic amount or a droplet of the allergen onto the patient's skin, as long as the location of the micro-incision coincides with the location where the allergen is deposited. Other commonly used tests are for example skin scratch tests: a deep dermic scratch is performed with help of the blunt bottom of a lancet and a droplet of an allergen is deposited on the deep dermic scratch, thereby bringing the allergen in contact with the body. Other commonly used tests are for example intradermic tests: a tiny quantity of allergen is injected under the dermis with a hypodermic syringe, thereby bringing the allergen in contact with the body. Other commonly used tests are for example skin scrape tests: a superficial scrape is performed with help of a needle to remove the superficial layer of the epidermis and a droplet of an allergen is deposited on the superficial scrape, thereby bringing the allergen in contact with the body. Other commonly used tests are for example patch tests wherein a patch is applied to the skin, where the patch contains the allergen.

Such allergen skin reaction tests allow testing multiple allergens on the forearm or on the back of a patient. However, the tests can easily become lengthy as the suitably trained medical staff must perform the pricks or punctures or scratches, or apply patches, individually for each separate allergen. The time needed to perform allergen skin reaction tests for several allergens can lead to discomfort for the patient. Additionally, errors can easily occur along this time-consuming process. Attention must indeed always be paid to the use of a sterile lancet for each perforation stitch to ensure that the test is unambiguous, in order to prevent corresponding cross-transfers of an allergen with another through contaminated lancets. Errors can also occur when mislabelling pricks or punctures or patches and their corresponding allergens, thereby jeopardizing the relevance of the tests.

Another limitation of these allergen skin reaction tests is that they all rely on the manual actuation of the medical personnel when pricking or puncturing the skin of the patient. Discrepancies in the penetration depths of the lancets can thereby be observed. For example, if the manual pressure when pricking is too low, there may be insufficient perforation of the uppermost skin layer and thus insufficient contact of the allergen with the immune system. On the other hand, deep perforation can lead to vascular injuries and corresponding bleeding, through which the allergen can be washed out of the perforation location, or through which the allergen undesirably comes into contact with the bloodstream.

In all cases where the allergen skin reaction test is positive, the skin will become raised in the form of a wheal, red and appear itchy at the location where the allergen came in contact with the body.

The results of skin prick tests are recorded manually by medical staff who manually annotates and/or manually measures and/or visually evaluates the size of the wheals - larger wheals indicating that the subject is more sensitive to that particular allergen. Detecting allergies is therefore performed in a laborious manner prone to errors.

### Summary

It is thus an object of embodiments of the present disclosure to propose a system and the related method for the automatic detection and metrology of an allergen skin reaction. More specifically, it is an object of embodiments of the present disclosure to propose a system and the related method for robustly and accurately perform allergen skin reaction tests for several allergens and for providing reliable results which will facilitate diagnosis of allergic reactions, thereby improving the accuracy of the detection of allergies.

The scope of protection sought for various embodiments of the present disclosure is set out by the independent claims.

The embodiments and features described in this specification that do not fall within the scope of the independent claims, if any, are to be interpreted as examples useful for understanding various embodiments of the present disclosure.

There is a need for minimizing errors in allergen skin reaction tests by removing manual actuation when performing the allergen skin reaction tests and when collecting the results of the allergen skin reaction tests.

Amongst others, it is an object of embodiments of the present disclosure to automate allergen skin reaction tests and the collection of allergen skin reaction tests results.

This object is achieved, according to a first example aspect of the present disclosure, by a system for detection and metrology of an allergen skin reaction of an individual, wherein the system comprises:
- testing means configured to host a body part of the individual and to subject a skin surface area of the body part to an allergen skin reaction test;
- illumination means configured to successively illuminate the skin surface area having been subjected to the allergen skin reaction test under one or more predetermined illumination angles with respect to acquisition means;
- the acquisition means fixedly positioned with respect to the skin surface area, wherein the acquisition means is configured to image, for each of the predetermined illumination angles, the illuminated skin surface; and
- analysing means configured to determine one or more illumination-angle-dependent variations in light intensity imaged by the acquisition means, wherein the illumination-angle-dependent variations are caused by one or more wheals on the skin surface area.

The system according to the present disclosure comprises illumination means which are configured to illuminate the skin surface area of the body part of the patient having been subjected to the allergen skin reaction test under one or more predetermined illumination angles. In other words, light is shined onto the skin surface area having been subjected to an allergen skin reaction test under one or more predetermined angles with respect to the acquisition means, and the system according to the present disclosure images the illuminated skin surface for each of the predetermined illumination angles. An image of the illuminated skin surface is therefore acquired by the system according to the present disclosure for each predetermined illumination angle. Subjecting the skin surface area to an allergen during an allergen skin reaction test might cause an allergic reaction at the position where the allergen skin reaction test was performed with this allergen. Usually, the allergic reaction comprises presence of a wheal on the skin surface area, i.e. a cutaneous condition as part of an allergic reaction. In the context of the present disclosure, a wheal is for example a rounded and/or flat-topped papule or plaque, which can be for example pale red, and that is characteristically evanescent, disappearing within 24 to 48 hours after the allergen skin reaction test. This temporary raised bubble of taut skin on the site of a properly delivered epicutaneous test can also be called a welt. Illumination of the skin surface area is swept for each predetermined illumination angles and the system according to the present disclosure images the illuminated skin surface for each predetermined illumination angles. As the acquisition means of the system according to the present disclosure are positioned at a fixed angle and distance with respect to the skin surface area during the imaging of the skin surface area for two or more different predetermined illumination angles, each wheal on the illuminated skin surface will cause a variation in the light intensity imaged by the system according to the present disclosure between the two or more different predetermined illumination angles. For example, raised papules will be lit by the illumination means and the shadow generated by the raised papules and captured by the acquisition means will change in function of the predetermined illumination angle under which the illumination means lit the skin surface area. The system according to the present disclosure then determines variations in the light intensity collected by the acquisition means between one predetermined illumination angle and one or more other predetermined illumination angles. When such variations in light intensity are determined by the system according to the present disclosure, presence of one or more wheals on the skin surface area is detected. The system according to the present disclosure images the skin surface area in black and white. Alternatively, the system according to the present disclosure images the skin surface area in colors.

The system according to the present disclosure allows the automatic testing and the automatic detection of allergen skin reaction, i.e. of an allergic reaction of the skin surface area of an individual to one or more allergens. The automatization of the process of testing, detecting, and evaluating the allergen skin reactions minimizes the manual and/or visual actions that were previously required with prior art solution in order to test and detect allergic skin reactions. Indeed, no human intervention is needed to perform the allergen skin reaction tests on the body part of the individual and no human intervention is needed to collect data on the reaction of the individual to the allergens from the skin surface area having been subjected to the allergen skin reaction test. Automatizing the allergen skin reaction tests ensures the reproducibility and the repeatability of the allergen skin reaction tests from one allergen to another and/or from one individual to another, in terms of incision as well as in terms of quantity of allergen disposed. With the system according to the present disclosure, the skin surface area of the individuals on which allergen skin reaction tests are performed will be punctured and/or incised in the same manner by the system, with uniform prick pressure, thereby ensuring good contact between the immune system and the allergen while preventing discrepancies in the penetration depths of the allergens and also guaranteeing the health of the patient by preventing unwanted bleeding and hazardous contact between the bloodstream of the patient and an allergen which could for example endanger the life of the patient. This makes the detection and the monitoring of allergic skin reaction with the system according to the present disclosure consistent and robust.

Additionally, minimizing human action in the testing and the detection of allergen skin reaction also renders the results unambiguous and considerably less error-prone than solutions of the prior art. The risk of mislabelling pricks or punctures and their corresponding allergens is minimized, thereby improving the relevance of the tests. The position of the pricks and/or of the corresponding allergens is automatically determined and saved by the system according to the present disclosure, thereby ensuring the alignment of the pricks and the allergens for optimum testing conditions. Cross-transfers of an allergen with another through contaminated test equipment is also unlikely with the system according to the present disclosure. The risk of performing an inaccurate and/or incomplete visual assessment of the reaction of the individual to one or more allergens by only looking at the skin surface area having been subjected to an allergen skin reaction test is minimized with the system according to the present disclosure. The system according to the present disclosure acquires and provides reliable and accurate data which helps the medical staff accurately detect, evaluate, measure, quantify the allergic reaction. The system according to the present disclosure therefore provides fast, error-free data comprising information indicative for presence of an allergic reaction to an allergen and information indicative for a degree of allergic sensitivity of the individual to this allergen, larger wheals indicating that the subject is more sensitive to that particular allergen. The data acquired and collected by the system according to the present disclosure facilitates the diagnosis of the medical staff of presence or absence of an allergic skin reaction and, if present, of the intensity of the allergic reaction.

With the system according to the present disclosure, and more particularly with the testing means of the system according to the present disclosure, it becomes possible to test reaction of an individual to several allergen skin reaction tests simultaneously and it also becomes possible to detect the reaction of an individual to several allergen skin reaction tests for example simultaneously using the same system. This makes the testing and the detection of allergen skin reactions more time efficient for the medical staff, but also for the individual being tested, thereby increasing his comfort with respect to prior art testing systems. Alternatively, it is also possible with the system according to the present disclosure to test skin reaction to one allergen at a time. This improves the comfort of the patient.

The reaction of the individual to each allergen skin reaction test performed on the skin surface area can be individually monitored by the system according to the present disclosure. Alternatively, the reaction of the individual to several allergens can be monitored simultaneously by the system according to the present disclosure, thereby rapidly and efficiently identifying allergens to which an individual is allergic and coming up with an individual-specific and customized treatment of these allergies.

A body part in the context of the present disclosure is for example the forearm of an individual, i.e. the region of the upper limb between the elbow and the wrist of the individual. The skin surface area of the body part in the context of the present disclosure is for example the anterior skin surface of the forearm of the individual, wherein the anterior skin surface of the forearm is usually being less hairy than the posterior skin surface of the forearm of the individual. Performing the allergen skin reaction tests on the forearm of the individual has a further advantage: the radial and ulnar arteries and their branches supply the blood to the forearm and they usually run on the anterior face of the forearm, thereby allowing to reliably test the immune system of the individual during the allergen skin reaction test.

Allergen skin reaction tests in the context of the present disclosure comprises a range of methods for medical diagnosis of allergies that attempts to provoke a small, controlled, allergic response. Typical methods of allergen skin reaction tests comprise introducing a microscopic amount of an allergen to a patient's skin by various means, for example on the forearm of the patient. Preferably, an allergen may be deposited in the form of a liquid droplet onto the skin surface area of the body part. Alternatively, an allergen may be deposited in another form onto the skin surface area of the body part. The most used allergen skin reaction tests are skin prick tests. For example, a droplet of an allergen is deposited on the skin surface area of the arm and then the skin surface area is pricked or punctured at the location of the droplet. Alternatively, the skin of for example the forearm of a patient is pricked or punctured with a needle or a pin containing a small amount of the allergen, thereby making a micro-incision on the skin of the patient and simultaneously bringing the allergen in contact with the body. Alternatively, making a micro-incision on the skin of a patient precedes or succeeds the deposition of a microscopic amount or a droplet of the allergen onto the patient's skin, as long as the location of the micro-incision coincides with the location where the allergen is deposited. Developing an automatic test allows to reduce the amount of expensive allergen needed to achieve results, thereby further reducing costs. Other commonly used allergen skin reaction tests are for example skin scratch tests: a deep dermic scratch is performed with help of the blunt bottom of a lancet and a droplet of an allergen is deposited on the deep dermic scratch, thereby bringing the allergen in contact with the body. Other commonly used allergen skin reaction tests are for example intradermic tests: a tiny quantity of allergen is injected under the dermis with a hypodermic syringe, thereby bringing the allergen in contact with the body. Other commonly used tests are for example skin scrape tests: a superficial scrape is performed with help of a needle to remove the superficial layer of the epidermis and a droplet of an allergen is deposited on the superficial scrape, thereby bringing the allergen in contact with the body. The system according to the present disclosure comprises testing means which are configured to subject a skin surface area of the patient to one or more of these allergen skin reaction tests.

Illumination means in the context of the present disclosure comprise one or more light sources. A light source is for example a lamp or a LED, or any suitable light source. For example, the illumination means comprise one or more lighting strips, such as for example LED strips. For example, the illumination means are an LED strip wherein the LED strip demonstrates a radiation angle of for example 120 degrees. The light intensity imaged by the acquisition means corresponds to light generated by the illumination means and reflected by the skin surface area. A predetermined illumination angle corresponds to the angle formed between the illumination means and the skin surface area of the patient. Variations in the light intensity collected by the acquisition means correspond to one or more changes in the light intensity imaged by the acquisition means between two or more predetermined illumination angles. These variations are therefore illumination-angle-dependent variations. The illuminated skin surface corresponds to the area of the skin surface area of the body part subjected to the allergen skin reaction test which is exposed to the illumination from the illumination means under a predetermined illumination angle. In other words, the illuminated skin surface is different for each predetermined illumination angle and the acquisition means are configured to image each illuminated skin surface for each corresponding predetermined illumination angle. For example, one or more light sources according to the present disclosure emit in the visible spectrum. The illumination means is configured to emit one or more wavelengths within the visible spectrum.

The acquisition means in the context of the present disclosure are for example a camera. Alternatively, the acquisition means are any suitable sensor configured to capture light, and comprise for example one or more photosensitive sensors, and/or one or more photosensitive cells, and/or one or more photodiodes, and/or one or more photoresistors, etc. A predetermined illumination angle is understood as the angle formed between the receiving direction along which the acquisition means are fixedly positioned and the direction substantially symmetrically dividing the illumination aperture or emission angle of the illumination means emitting the illumination, such as for example the light source emitting the illumination. For example, the acquisition means according to the present disclosure comprises one or more red-blue-green detectors.

The system according to the present disclosure may further comprise a housing configured to host the testing means and the illumination means. Optionally, the acquisition means may be also comprised in the housing. Alternatively, the acquisition means are not integrally combined in the same housing with the testing means, the illumination means and/or the analysing means. Optionally, the analysing means may be also comprised in the housing. Alternatively, the analysing means are not integrally combined in the same housing with the testing means, the illumination means and/or the acquisition means. For example, the analysing means may be a remote computer, tablet or smartphone, operationally coupled to the testing means, the illumination means and/or the acquisition means.

The system according to the present disclosure optionally comprises a motor configured to displace the illumination means. The motor may cause the illumination means to rotate around the skin surface area in a steady speed, thus allowing an even sweeping of the illumination of the skin surface area from one predetermined illumination angle to another. Alternatively, the system according to the present disclosure has predetermined illumination angles for the illumination means registered and the motor is configured to displace the illumination means to these predetermined illumination angles sequentially. The system according to the present disclosure optionally comprises a motor configured to displace the acquisition means. The motor may cause the acquisition means to translate above the skin surface area along a traverse direction in a steady speed until the acquisition means reach a position for which the clear image of the skin surface area can be captured by the acquisition means. Alternatively, the system according to the present disclosure has predetermined positions for the acquisition means registered and the motor is configured to displace the acquisition means, along a receiving direction traverse to the longitudinal direction along which the arm extends, to these predetermined positions sequentially until the acquisition means reach a position for which the clear image of the skin surface area can be captured by the acquisition means.

According to example embodiments:
- the skin surface area extends along a longitudinal direction;
- the acquisition means are fixedly positioned with respect to the skin surface area along a receiving direction traverse to the longitudinal direction;
- the predetermined illumination angles are formed between the receiving direction and a lighting direction of the illumination means, wherein the illumination means tangentially illuminates the skin surface area along the lighting direction.

A predetermined illumination angle in the context of the present disclosure is understood as the angle formed between the receiving direction along which the acquisition means are fixedly positioned, and the lighting direction of the illumination means. The lighting direction of the illumination means in the context of the present disclosure corresponds to the direction along which the illumination means tangentially illuminates the skin surface area of the body part. For example, the lighting direction substantially symmetrically divides the illumination aperture or the emission angle of the illumination means emitting the illumination and the illumination means tangentially illuminates the skin surface area of the body part along this lighting direction. For example, when the illumination means comprises a light source, the lighting direction is the direction along which the illumination means tangentially illuminates the skin surface area of the body part and the direction which substantially symmetrically divides the illumination aperture or the emission angle of the light source. In other words, the illumination means is positioned at an illumination position along the lighting direction, and the illumination means illuminates the skin surface area tangentially along the lighting direction such that a predetermined illumination angle is formed between the lighting direction and the receiving direction. Each of the one or more predetermined illumination angles of the present disclosure is therefore formed between the receiving direction along which the acquisition means are positioned and the lighting direction of the illumination means, wherein the illumination means tangentially illuminates the skin surface area along the lighting direction and wherein the lighting direction substantially symmetrically divides the illumination aperture or emission angle of the illumination means. When the illumination means comprises one light source, each of the one or more predetermined illumination angles of the present disclosure is formed between the receiving direction along which the acquisition means are positioned and the lighting direction of the light source, wherein the light source tangentially illuminates the skin surface area along the lighting direction and wherein the lighting direction substantially symmetrically divides the illumination aperture or emission angle of the light source. When the illumination means comprises several light sources, each of the one or more predetermined illumination angles of the present disclosure is formed between the receiving direction along which the acquisition means are positioned and the lighting direction of one of the light sources, wherein this light source tangentially illuminates the skin surface area along the lighting direction and wherein the lighting direction substantially symmetrically divides the illumination aperture or emission angle of this light source. The illumination means may for example be mounted on one or more mounting supports such that, the illumination means may tangentially illuminate the skin surface area under more than one predetermined illumination angles. For example, when the illumination means are fixedly mounted on one or more mounting supports, i.e. when the illumination means are at a fixed illumination position, the illumination means may still rotate around the fixed illumination position to illuminate the skin surface area under several lighting directions such that the illumination means illuminate the skin surface area under several predetermined illumination angles formed between the receiving direction and each of the lighting directions. Alternatively, when the illumination means are mounted on one or more mounting supports and may be positioned at several illumination positions, the illumination means illuminate the skin surface area under several predetermined illumination angles formed between the receiving direction and each of the lighting directions at each of the illuminated positions. Alternatively, when the illumination means are mounted on one or more mounting supports and may be positioned at several illumination positions, the illumination means may still rotate around each illumination position to illuminate the skin surface area under several lighting directions such that the illumination means illuminate the skin surface area under several predetermined illumination angles formed between the receiving direction and each of the lighting directions at each of the illuminated positions. Alternatively, when the illumination means are mounted on one or more mounting supports and may be positioned at several illumination positions, the illumination means may still rotate around each illumination position and/or translate to another illumination position to illuminate the skin surface area under several lighting directions such that the illumination means illuminate the skin surface area under several predetermined illumination angles formed between the receiving direction and each of the lighting directions at each of the illuminated positions.

According to example embodiments, the acquisition means are further configured to capture one or more images of the skin surface area for each of the predetermined illumination angles, thereby generating a set of images of the skin surface area for the allergen skin reaction test.

For each illumination angle, an image of the illuminated skin surface is captured by the acquisition. Sweeping the illumination at the predetermined illumination angles thereby results in a set of images. Each image acquired by the acquisition means comprises an imaged light intensity which corresponds to the light generated by the illumination means which is reflected by the skin surface area. By comparing the light intensity captured under one predetermined illumination angle to the light intensity captured under another and different predetermined illumination angle, i.e. by comparing the light intensity between at least two images of the set of images, the system is configured to determined one or more changes in light intensity between the two predetermined illumination angles. An image acquired by the acquisition means is for example a picture or a photography of the skin surface area under one predetermined illumination angle. Alternatively, an image acquired by the acquisition means is for example a frame of a video of the skin surface area acquired by the acquisition means under one predetermined illumination angle. Alternatively, an image acquired by the acquisition means is any two-dimensional representation of the skin surface area under one predetermined illumination angle. One or more images of the skin surface area comprise in black and white. Alternatively, one or more images of the skin surface area comprise colors. Alternatively, one or more images of the skin surface area comprise black and white and one or more images of the same skin surface area comprise colors.

According to example embodiments, the analysing means are further configured to process the set of images and to determine, from the set of images, one or more of the following:
- reflections generated from the illumination by the wheals on the skin surface area;
- shadows generated from the illumination by the wheals on the skin surface area; thereby determining the illumination-angle-dependent variations in light intensity imaged by the acquisition means.

Each image acquired by the acquisition means comprises an imaged light intensity which corresponds to the light generated by the illumination means which is reflected by the skin surface area. Analysing light patterns on the skin surface area allows to detect presence of one or more wheals on the skin surface area. When a wheal is present on the skin surface area, light of the illumination means will be reflected by the skin and by the wheal, and a shadow of the wheal will be formed on the skin surface area, wherein the shape and the position of the shadow are dependent upon the predetermined illumination angle. By comparing the light intensity captured under one predetermined illumination angle to the light intensity captured under another and different predetermined illumination angle, i.e. by comparing the light intensity of the reflected light between at least two images of the set of images, and by comparing the variations or changes of the shadows imaged between at least two images of the set of images, the system is configured to determined one or more changes in light intensity between the two predetermined illumination angles.

The skin surface area is illuminated under one or more predetermined illumination angles. For each predetermined illumination angle, the illumination means tangentially illuminates the skin surface area along a lighting direction. The acquisition means image the skin surface area being illuminated under each predetermined illumination angle. Reflections will be detected by the acquisition means, wherein the reflections correspond to the illumination of the illumination means being reflected by the skin surface area. Shadows will be detected by the acquisition means, wherein the shadows correspond to the illumination of the illumination means not being reflected by the skin surface area. The skin surface area facing the illumination means until the lighting direction may reflect the illumination of the illumination means, thereby causing reflections which will be imaged by the acquisition means. Wheals on the skin surface area facing the illumination means until the lighting direction may reflect the illumination of the illumination means, thereby generating reflections and shadows which will be imaged and generate illumination-angle-dependent variations in light intensity determined by the acquisition means from the set of images for the predetermined illumination angles. The skin surface area facing away from the illumination means from the lighting direction may not reflect the illumination of the illumination means, thereby causing shadows which will be imaged by the acquisition means.

According to example embodiments, the analysing means are further configured to determine a location of the wheals on the skin surface area using the illumination-angle-dependent variations in light intensity imaged by the acquisition means.

It becomes possible to automatically determine the presence and the position of wheals on the skin surface area with the system. Using the variations of the reflected light formed by the illumination of the skin surface area when sweeping the illumination over the predetermined illumination angles, such as for example the shadows, the system can identify the position of the wheals. This way, the system can identify to which allergen corresponds the wheal detected on the skin surface area. The system has preliminary knowledge about the position of the pricks and/or the position where the allergens were deposited. When the location of a wheal on the skin surface area corresponds to the known position of a prick and/or to the known position of an allergen, the system can reliably and accurately identify to which allergen the individual reacts and is likely to be allergic. Identifying the position of wheals on the skin surface area is therefore done automatically without relying on manual and/or visual inspection of the skin surface area.

According to example embodiments, the analysis means are further configured to determine, from the set of images, a size and/or a shape of one or more of the wheals on the skin surface area.

This way, the system, more particularly the analysing means, may comprise measuring the dimensions of the wheals, thereby automatizing the metrology of the detection of allergies. The system may comprise comparing the measured dimensions of a wheal, such as for example its size and/or shape, with one or more referenced ranges of dimensions for wheals, wherein a referenced range of dimensions may be indicative for a degree of sensitivity to an allergen. This way, when the dimensions of a wheal fall within a referenced range of dimensions, it becomes possible to determine a degree of sensitivity of the individual to the corresponding allergen. Alternatively, the system may comprise comparing the measured dimensions of a wheal, such as for example its size and/or shape, with the measured dimensions of another wheal formed on the same skin surface area, thereby determining a relative degree of sensitivity of the individual to the corresponding allergens.

According to example embodiments, the acquisition means are positioned along the receiving direction traverse to the longitudinal direction to image the illuminated skin surface under each of the predetermined illumination angles.

This way, the acquisition means faces the skin surface area for each of the predetermined illumination angles. The acquisition means can then capture an image of the entire skin surface area for all the predetermined illumination angles.

According to example embodiments, the acquisition means are positioned at a fixed predetermined receiving distance from the skin surface area for all of the predetermined illumination angles.

This way, the acquisition means remain fixedly positioned facing the skin surface area for each of the predetermined illumination angles. This allows the system to compare the different light patterns, i.e. the variations in the light intensity captured by the acquisition means such as the reflected light and/or the shadows, between the different images of a set of images.

According to example embodiments, the illumination means comprise one or more light sources, wherein each light source is configured to illuminate the skin surface area; and wherein the system further comprises one or more mounting supports configured to hold the one or more light sources.

This way, illumination of the skin surface area under the predetermined illumination angles may be achieved in different ways. For example, one light source can be repositioned around the body part and illuminate the skin surface area under each of the predetermined illumination angles, either by continuously emitting, or by pulsed emission, or by emitting only when positioned at the predetermined illumination angles. This way, only one light source is necessary, thereby saving costs. Alternatively, the system comprises a plurality of light sources, wherein each light source remains fixedly positioned at a predetermined illumination angle and wherein the illumination means illuminate the skin surface area with a one light source at a time or according to a predetermined illumination sequence. A predetermined illumination angle is understood as the angle formed between the receiving direction along which the acquisition means are fixedly positioned and the direction symmetrically dividing the emission angle of the light source emitting the illumination.

According to example embodiments, the one or more light sources and/or the mounting supports are further configured to rotate around the body part in a plane comprising the receiving direction and traverse to the longitudinal direction such that the one or more light sources illuminate the skin surface area under the one or more predetermined illumination angles.

For example, one light source is mounted on one mounting support which can rotate, or at least partially rotate, around the body part such that the one light source illuminates the skin surface area under each of the predetermined illumination angles. Alternatively, several light sources are mounted on one or more mounting supports which can rotate, or at least partially rotate, around the body part such that one or more light sources illuminate the skin surface area under each of the predetermined illumination angles.

According to example embodiments, the light sources and/or the mounting supports are further configured to rotate around the body part in a plane comprising the longitudinal direction of the body part and the receiving direction such that the one or more light sources illuminate the skin surface area under the one or more predetermined illumination angles.

For example, one light source is mounted on one mounting support which can rotate, or at least partially rotate, around the body part such that the one light source illuminates the skin surface area under each of the predetermined illumination angles. Alternatively, several light sources are mounted on one or more mounting supports which can rotate, or at least partially rotate, around the body part such that one or more light sources illuminate the skin surface area under each of the predetermined illumination angles.

According to example embodiments, the testing means is configured to simultaneously deposit one allergen at a predetermined position on the skin surface area and incise the skin surface area of the individual at each of the predetermined positions.

The testing means may select a point in the skin surface area, providing an allergen on the point and performing an incision on the point. A plurality of incisions may be made and combined with a plurality of allergens with only one system according to the present disclosure.

According to example embodiments, the testing means may further comprise a clamp configured to maintain the body part immobile when imaging the illuminated skin surface for each of the predetermined illumination angles.

This way, the clamp maintains the body part in a fixed position during the allergen skin reaction test and/or during the imaging of the illuminated skin surface under the predetermined illumination angles. This prevents the system from performing the allergen skin reaction test twice at the same location or from confusing the location of an allergen skin reaction test with another, thereby guaranteeing the accuracy of the analysis. Additionally, the allergens may be released in a controlled manner on an immobile skin surface area, thereby avoiding spillage of the allergens and contamination of areas of subsequent incisions that could affect the allergen skin reaction test. The clamp may be ergonomically adjusted to allow the individual to rest the body part immobile, or substantially immobile, or in a substantially stationary position, for an amount of time that is required to perform the allergen skin reaction test and/or for an amount of time that is required to image the skin surface area under the predetermined illumination angles.

Optionally, the system further comprises a handle to facilitate the maintenance of the substantially stationary or substantially immobile position of the body part during the allergen skin reaction test and/or during acquisition of the set of images for the predetermined illumination angles.

Optionally, the system further comprises an allergen storing unit configured to store one or more allergens, each allergen being stored in an individual and isolated compartment. Conservations of the allergens in the storing unit may be thermally controlled to preserve their integrity.

According to a second aspect of the present disclosure, there is provided a method for detection and metrology of an allergen skin reaction of an individual, wherein the method comprises the steps of:
- hosting a body part of the individual;
- subjecting a skin surface area of the body part to an allergen skin reaction test;
- successively illuminating the skin surface area having been subjected to the allergen skin reaction test under one or more predetermined illumination angles with respect to a fixed position;
- imaging the illuminated skin surface from the fixed position with respect to the skin surface area and for each of the predetermined illumination angles; and
- determining one or more illumination-angle-dependent variations in light intensity being imaged, wherein the illumination-angle-dependent variations are caused by one or more wheals on the skin surface area.

The method according to the present disclosure comprises illuminating the skin surface area of the body part of the patient having been subjected to the allergen skin reaction test under one or more predetermined illumination angles. In other words, light is shined onto the skin surface area having been subjected to an allergen skin reaction test under one or more predetermined angles with respect to the acquisition means, and the method according to the present disclosure images the illuminated skin surface for each of the predetermined illumination angles. An image of the illuminated skin surface is therefore acquired for each predetermined illumination angle. Subjecting the skin surface area to an allergen during an allergen skin reaction test might cause an allergic reaction at the position where the allergen skin reaction test was performed with this allergen. Usually, the allergic reaction comprises presence of a wheal on the skin surface area, i.e. a cutaneous condition as part of an allergic reaction. In the context of the present disclosure, a wheal is for example a rounded and/or flat-topped papule or plaque, which can be for example pale red, and that is characteristically evanescent, disappearing within 24 to 48 hours after the allergen skin reaction test. This temporary raised bubble of taut skin on the site of a properly delivered epicutaneous can also be called a welt. Illumination of the skin surface area is swept for each predetermined illumination angles and the illuminated skin surface is imaged for each predetermined illumination angles. As the images of the skin surface area for two or more different predetermined illumination angles are acquired from a fixed position, i.e. at a fixed angle and distance with respect to the skin surface area, each wheal on the illuminated skin surface will cause a variation in the light intensity imaged between the two or more different predetermined illumination angles. For example, raised papules will be lit and the shadow generated by the raised papules and captured with the method according to the present disclosure will change in function of the predetermined illumination angle under which the skin surface area is lit. The method according to the present disclosure then determines variations in the light intensity collected between one predetermined illumination angle and one or more other predetermined illumination angles. When such variations in light intensity are determined by the method according to the present disclosure, presence of one or more wheals on the skin surface area is detected.

The method according to the present disclosure allows the automatic testing and the automatic detection of allergen skin reaction, i.e. of an allergic reaction of the skin surface area of an individual to one or more allergens. The automatization of the process of testing, detecting, and evaluating the allergen skin reactions minimizes the manual and/or visual actions that were previously required with prior art solution in order to test and detect allergic skin reactions. Indeed, no human intervention is needed to perform the allergen skin reaction tests on the body part of the individual and no human intervention is needed to collect data on the reaction of the individual to the allergens from the skin surface area having been subjected to the allergen skin reaction test. Automatizing the allergen skin reaction tests ensures the reproducibility and the repeatability of the allergen skin reaction tests from one allergen to another and/or from one individual to another, in terms of incision as well as in terms of quantity of allergen disposed. With the method according to the present disclosure, the skin surface area of the individuals on which allergen skin reaction tests are performed will be punctured and/or incised in the same manner, with uniform prick pressure, thereby ensuring good contact between the immune system and the allergen while preventing discrepancies in the penetration depths of the allergens and also guaranteeing the health of the patient by preventing unwanted bleeding and hazardous contact between the bloodstream of the patient and an allergen which could endanger the life of the patient. This makes the detection and the monitoring of allergic skin reaction with the method according to the present disclosure consistent and robust.

Additionally, minimizing human action in the testing and the detection of allergen skin reaction also renders the results unambiguous and considerably less error-prone than solutions of the prior art. The risk of mislabelling pricks or punctures and their corresponding allergens is minimized, thereby improving the relevance of the tests. The position of the pricks and/or of the corresponding allergens is automatically determined and saved by the method according to the present disclosure, thereby ensuring the alignment of the pricks and the allergens for optimum testing conditions. Cross-transfers of an allergen with another through contaminated test equipment is also unlikely with the method according to the present disclosure. The risk of performing an inaccurate and/or incomplete visual assessment of the reaction of the individual to one or more allergens by only looking at the skin surface area having been subjected to an allergen skin reaction test is minimized with the method according to the present disclosure. The method according to the present disclosure acquires and provides reliable and accurate data which helps the medical staff accurately detect, evaluate, measure, quantify the allergic reaction. The method according to the present disclosure therefore provides fast, error-free data comprising information indicative for presence of an allergic reaction to an allergen and information indicative for a degree of allergic sensitivity of the individual to this allergen, larger wheals indicating that the subject is more sensitive to that particular allergen. The data acquired and collected by the method according to the present disclosure facilitates the diagnosis of the medical staff of presence or absence of an allergic skin reaction and, if present, of the intensity of the allergic reaction.

According to example embodiments, the method further comprises the steps of:
- capturing one or more images of the skin surface area for each of the predetermined illumination angles, thereby generating a set of images of the skin surface area for the allergen skin reaction test;
- determining on the set of images one or more illumination-angle-dependent variations in light intensity being imaged, wherein the illumination-angle-dependent variations are caused by one or more wheals on the skin surface area, thereby detecting presence of one or more wheals on the skin surface area; and
- determining, from the set of images, a location and/or a size and/or a shape of the wheals on the skin surface area.

This way, the method comprises calibration steps when the skin surface area is imaged while not illuminated. Optionally, when the illumination means comprise several light sources wherein each light source is positioned to illuminate the skin surface area under a predetermined illumination angle, the method further comprises capturing one or more images of the skin surface area having been subjected to the allergen skin reaction when the skin surface area is illuminated under several of the predetermined illumination angles available. Alternatively, the method further comprises the step of capturing one or more images of the skin surface area having been subjected to the allergen skin reaction when the skin surface area is not illuminated.

According to example embodiments, the method further comprises the steps of simultaneously depositing one allergen at a predetermined position on the skin surface area and incising the skin surface area of the individual at each of the predetermined positions.

According to a third aspect of the present disclosure, there is provided a computer program product comprising computer-executable instructions for causing a system to perform at least the following:
- successively illuminating a skin surface area of a body part having been subjected to an allergen skin reaction test under one or more predetermined illumination angles with respect to a fixed position;
- imaging said illuminated skin surface from said fixed position with respect to said skin surface area and for each of said predetermined illumination angles; and
- determining one or more illumination-angle-dependent variations in light intensity being imaged, wherein said illumination-angle-dependent variations are caused by one or more wheals on said skin surface area.

According to a fourth aspect of the present disclosure, there is provided a computer readable storage medium comprising computer-executable instructions for performing the following steps when the program is run on a computer:
- successively illuminating a skin surface area of a body part having been subjected to an allergen skin reaction test under one or more predetermined illumination angles with respect to a fixed position;
- imaging said illuminated skin surface from said fixed position with respect to said skin surface area and for each of said predetermined illumination angles; and
- determining one or more illumination-angle-dependent variations in light intensity being imaged, wherein said illumination-angle-dependent variations are caused by one or more wheals on said skin surface area.

### Brief Description of the Drawings

Some example embodiments will now be described with reference to the accompanying drawings.
Fig. 1 depicts an example embodiment of a system according to the present disclosure.
Figs. 2A and 2B depict an example embodiment of illumination-angle-dependent variations in light intensity according to the present disclosure.
Figs. 3A and 3B depict example embodiments of a rotation of the illumination means of a system according to the present disclosure.
Fig. 4 shows an example embodiment of a suitable computing system for performing one or several steps in embodiments of the invention.

### Detailed Description of Embodiment(s)

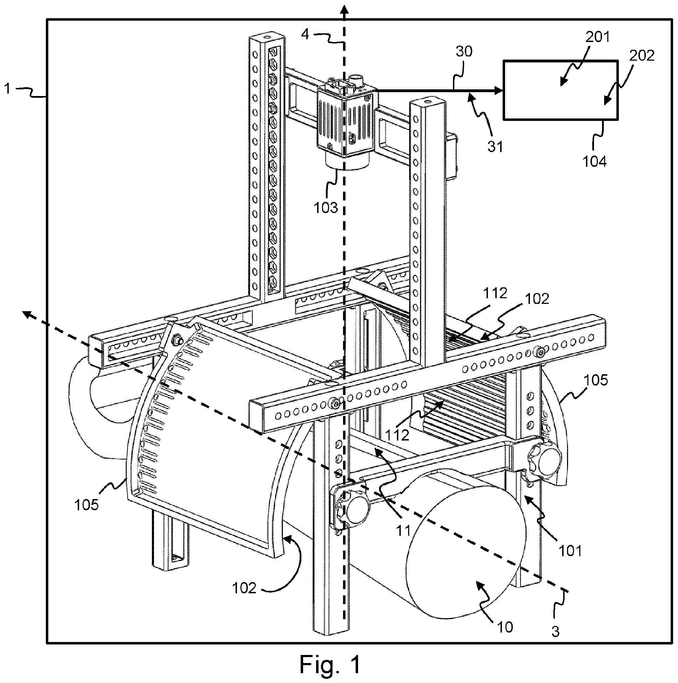

Fig. 1 illustrates an example embodiment of a system 1 according to the present disclosure for detection and metrology of an allergen skin reaction of an individual. The system 1 comprises testing means 101, illumination means 102, acquisition means 103 and analyzing means 104. The testing means 101 are configured to host a body part 10 of the individual and to subject a skin surface area 11 of the body part 10 to an allergen skin reaction test. For the sake of clarity of the drawing, Fig. 1 does not illustrate the skin surface area 11 being subjected to the allergen skin reaction test. The illumination means 102 are configured to successively illuminate the skin surface area 11 having been subjected to the allergen skin reaction test under one or more predetermined illumination angles with respect the acquisition means 103. The acquisition means 103 are fixedly positioned with respect to the skin surface area 11. The acquisition means 103 are configured to image, for each of the predetermined illumination angles 20, the illuminated skin surface corresponding to the skin surface area 11 being illuminated by the illumination means 102 under a predetermined illumination angle. The acquisition means 103 capture one or more images 30 of the skin surface area 11 for each of the predetermined illumination angles, thereby generating a set of images 31 of the skin surface are 11 for the allergen skin reaction test. The analysing means 104 are configured to determine one or more illumination-angle-dependent variations 202 in light intensity 201 imaged by the acquisition means 103, wherein the illumination-angle-dependent variations 202 of the light intensity 201 are caused by one or more wheals on the skin surface area 11. The analysing means 104 are configured to process the set of images 31 to determine, from the set of images 31, reflections generated from the illumination by wheals on the skin surface area 11, and/or shadows generated from the illumination by wheals on the skin surface area 11, thereby determining the illumination-angle-dependent variations 202 in light intensity 201 imaged by the acquisition means 103. The analysing means 104 determine a location of the wheals on the skin surface area 11 using the illumination-angle-dependent variations 202 in light intensity 201 imaged by the acquisition means 103. The analysing means 104 are configured to determine, from the set of images 31, a size and/or a shape of one or more of the wheals on the skin surface area 11. The body part extends along a longitudinal direction 3 and the acquisition means 103 are positioned along a receiving direction 4 traverse to the longitudinal direction 3 to image the illuminated skin surface 21 under each predetermined illumination angle. The acquisition means 103 are positioned at a fixed predetermined receiving distance from the skin surface area 11 for all of the predetermined illumination angles. The illumination means 102 comprise one or more light sources 112, wherein each light source 112 is configured to illuminate the skin surface area 11. The system 1 further comprises one or more mounting supports 105 configured to hold the one or more light sources 112. The light sources 112 and/or the mounting supports 105 are configured to rotate around the body part 10 in a plane comprising the receiving direction 4 and traverse to the longitudinal direction 3 such that the light sources 112 illuminate the skin surface area 11 under the predetermined illumination angles. The light sources 112 and/or the mounting supports 105 are configured to rotate around the body part 10 in a plane comprising the receiving direction 4 and the longitudinal direction 3 such that the light sources 112 illuminate the skin surface area 11 under the predetermined illumination angles. The testing means 101 are configured to simultaneously deposit one allergen at a predetermined position on the skin surface area 11 and incise the skin surface area 11 at each of the predetermined positions. The testing means 101 optionally comprise a clamp which is not depicted on Fig. 1 for clarity and which maintains the body part 10 substantially immobile when imaging the illuminated skin surface for each of the predetermined illumination angles.

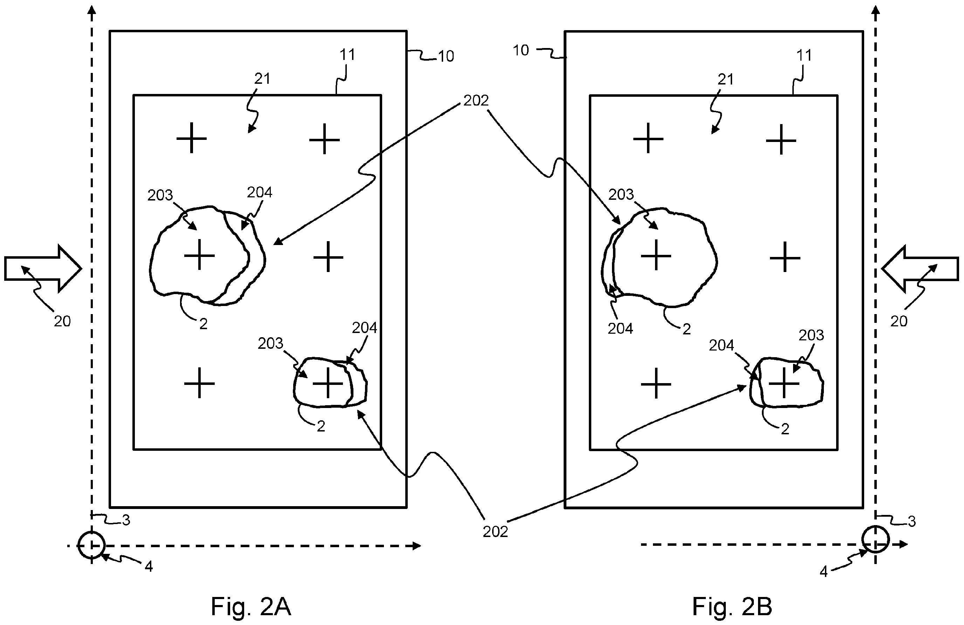

Figs. 2A and 2B depict an example embodiment of illumination-angle-dependent variations in light intensity according to the present disclosure. Components demonstrating an identical reference number than on Fig. 1 fulfill the same function. On Fig. 2A, a skin surface area 11 of a body part 10 extending along a longitudinal direction 3 is illuminated under a first predetermined illumination angle 20, thereby forming an illuminated skin surface 21. The skin surface area 11 has been subjected beforehand to an allergen skin reaction test, during which 6 allergens have been tested, as indicated by the crosses on Fig. 2A. Two wheals 2 are formed on the skin surface 11 and become visible on the illuminated skin surface 21. Each wheal 2 generates a reflection 203 on the illuminated skin surface 21 of the illumination under the first predetermined illumination angle 20 and each wheal 2 also generates a shadow on the illuminated skin surface 21 of the illumination under the first predetermined illumination angle 20. The same skin surface area 11 is now illuminated on Fig. 2B under a second predetermined illumination angle 20, thereby forming an illuminated skin surface 21. The predetermined illumination angles 20 are formed between a receiving direction 4 and a lighting direction of the illumination means, wherein the illumination means tangentially illuminates the skin surface area 11 along the lighting direction. The two wheals 2 previously visible on the illuminated skin surface 21 being illuminated under the first predetermined illumination angle 20, are still visible on the illuminated skin surface being illuminated under the second illumination angle 20. Under the second illumination angle 20, each wheal 2 generates a reflection 203 on the illuminated skin surface 21 of the illumination under the second predetermined illumination angle 20 and each wheal 2 also generates a shadow on the illuminated skin surface 21 of the illumination under the second predetermined illumination angle 20. The reflections 203 of the wheals 2 generated under the first predetermined illumination angle 20 and under the second predetermined illumination angle 20 are different. The shadows 204 of the wheals 2 generated under the first predetermined illumination angle 20 and under the second predetermined illumination angle 20 are different. The acquisition means of the system according to the present disclosure images the illuminated skin surface 21 of Fig. 2A illuminated under the first predetermined illumination angle 20 and further images the illuminated skin surface 21 of Fig. 2B illuminated under the second predetermined illumination angle 20. The analyzing means of the system according to the present disclosure then determines several illumination-angle-dependent variations 202 in the light intensity imaged by the acquisition means, wherein the illumination-angle-dependent variations 202 are caused by the two wheals 2 on the skin surface area 11. The analyzing means of the system according to the present disclosure therefore detect presence of the two wheals 2 on the skin surface area 11.

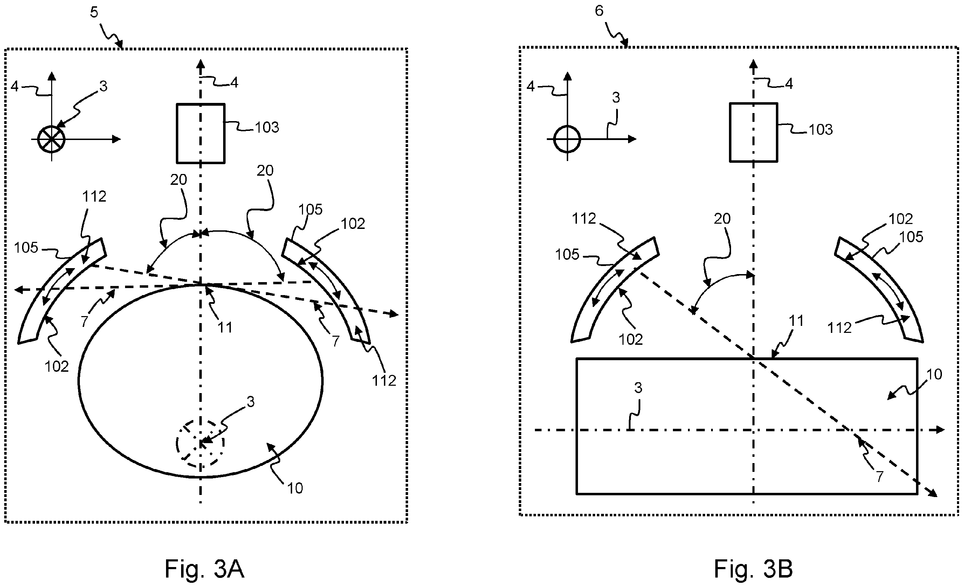

Figs. 3A and 3B depict example embodiments of a rotation of the illumination means of a system according to the present disclosure. Components demonstrating an identical reference number than on Fig. 1 or Figs. 2A or 2B fulfill the same function. The illumination means 102 are configured to successively illuminate the skin surface area 11 having been subjected to the allergen skin reaction test under one or more predetermined illumination angles 20 with respect the acquisition means 103. The acquisition means 103 are fixedly positioned with respect to the skin surface area 11. The acquisition means 103 are configured to image, for each of the predetermined illumination angles 20, the illuminated skin surface 21 corresponding to the skin surface area 11 being illuminated by the illumination means 102 under a predetermined illumination angle 30. The acquisition means 103 capture one or more images 30 of the skin surface area 11 for each of the predetermined illumination angles 20, thereby generating a set of images 31 of the skin surface are 11 for the allergen skin reaction test. Fig. 3A depicts illumination under two predetermined illumination angles 20, but these illuminations are understood to happen consecutively in the context of the present disclosure. The analysing means are configured to determine one or more illumination-angle-dependent variations in light intensity imaged by the acquisition means 103, wherein the illumination-angle-dependent variations of the light intensity are caused by one or more wheals on the skin surface area 11. The analysing means are configured to process the set of images to determine, from the set of images, reflections generated from the illumination by wheals on the skin surface area 11, and/or shadows generated from the illumination by wheals on the skin surface area 11, thereby determining the illumination-angle-dependent variations in light intensity imaged by the acquisition means 103. The analysing means determine a location of the wheals on the skin surface area 11 using the illumination-angle-dependent variations in light intensity imaged by the acquisition means 103. The analysing means are configured to determine, from the set of images, a size and/or a shape of one or more of the wheals on the skin surface area 11. The body part extends along a longitudinal direction 3 and the acquisition means 103 are positioned along a receiving direction 4 traverse to the longitudinal direction 3 to image the illuminated skin surface 21 under each predetermined illumination angle 20. The acquisition means 103 are positioned at a fixed predetermined receiving distance from the skin surface area 11 for all of the predetermined illumination angles. The illumination means 102 comprise one or more light sources 112, wherein each light source 112 is configured to illuminate the skin surface area 11. The predetermined illumination angles 20 are formed between the receiving direction 4 and a lighting direction 7 of the illumination means 102, wherein the illumination means 102 tangentially illuminates the skin surface area 11 along the lighting direction 7. A predetermined illumination angle 20 is understood as the angle formed between the receiving direction 4 along which the acquisition means 103 are fixedly positioned, and the lighting direction 7 of the illumination means 102. The lighting direction 7 of the illumination means 102 corresponds to the direction along which the illumination means 102 tangentially illuminates the skin surface area 11 of the body part 10. For example, the lighting direction 7 substantially symmetrically divides the illumination aperture or the emission angle of the illumination means 102 emitting the illumination and the illumination means 102 tangentially illuminates the skin surface area 11 of the body part 10 along this lighting direction 7. For example, when the illumination means 102 comprises a light source 112, the lighting direction 7 is the direction along which the illumination means 102 tangentially illuminates the skin surface area 11 of the body part 10 and the direction which substantially symmetrically divides the illumination aperture or the emission angle of the light source 112. In other words, the illumination means 102 is positioned at an illumination position along the lighting direction 7, and the illumination means 102 illuminates the skin surface area 11 tangentially along the lighting direction 7 such that a predetermined illumination angle 20 is formed between the lighting direction 7 and the receiving direction 4. Each of the one or more predetermined illumination angles 20 is therefore formed between the receiving direction 4 along which the acquisition means 102 are positioned and the lighting direction 7 of the illumination means 102, wherein the illumination means 102 tangentially illuminates the skin surface area 11 along the lighting direction 7 and wherein the lighting direction 7 substantially symmetrically divides the illumination aperture or emission angle of the illumination means 102. When the illumination means 102 comprises one light source 112, each of the one or more predetermined illumination angles 20 is formed between the receiving direction 4 along which the acquisition means 103 are positioned and the lighting direction 7 of the light source 112, wherein the light source 112 tangentially illuminates the skin surface area 11 along the lighting direction 7 and wherein the lighting direction 7 substantially symmetrically divides the illumination aperture or emission angle of the light source 112. When the illumination means 102 comprises several light sources 112, each of the one or more predetermined illumination angles 20 is formed between the receiving direction 4 along which the acquisition means 103 are positioned and the lighting direction 7 of one of the light sources 112, wherein this light source 112 tangentially illuminates the skin surface area 11 along the lighting direction 7 and wherein the lighting direction 7 substantially symmetrically divides the illumination aperture or emission angle of this light source 112. The illumination means 102 may for example be mounted on one or more mounting supports 105 such that, the illumination means 102 may tangentially illuminate the skin surface area 11 under more than one predetermined illumination angles 20. For example, when the illumination means 102 are fixedly mounted on one or more mounting supports 105, i.e. when the illumination means 102 are at a fixed illumination position, the illumination means 102 may still rotate around the fixed illumination position to illuminate the skin surface area 11 under several lighting directions 7 such that the illumination means 102 illuminate the skin surface area 11 under several predetermined illumination angles 20 formed between the receiving direction 4 and each of the lighting directions 7. Alternatively, when the illumination means 102 are mounted on one or more mounting supports 105 and may be positioned at several illumination positions, the illumination means 102 illuminate the skin surface area 11 under several predetermined illumination angles 20 formed between the receiving direction 4 and each of the lighting directions 7 at each of the illuminated positions. Alternatively, when the illumination means 102 are mounted on one or more mounting supports 105 and may be positioned at several illumination positions, the illumination means 102 may still rotate around each illumination position to illuminate the skin surface area 11 under several lighting directions 7 such that the illumination means 102 illuminate the skin surface area 11 under several predetermined illumination angles 20 formed between the receiving direction 4 and each of the lighting directions 7 at each of the illuminated positions. The system further comprises one or more mounting supports 105 configured to hold the one or more light sources 112. On Fig. 3A, the light sources 112 and/or the mounting supports 105 are configured to rotate around the body part 10 in a plane 5 comprising the receiving direction 4 and traverse to the longitudinal direction 3 such that the light sources 112 illuminate the skin surface area 11 under the predetermined illumination angles 20. On Fig. 3B, the light sources 112 and/or the mounting supports 105 are configured to rotate around the body part 10 in a plane 6 comprising the receiving direction 4 and the longitudinal direction 3 such that the light sources 112 illuminate the skin surface area 11 under the predetermined illumination angles 20. According to an alternative embodiment, one or more of the mounting supports 105 are straight and extending along the receiving direction 4. According to an alternative embodiment, one or more of the mounting supports 105 are straight and extending along the longitudinal direction 3. According to an alternative embodiment, one or more of the mounting supports 105 are straight and extending along the longitudinal direction 3 and one or more of the mounting supports 105 are straight and extending along the longitudinal direction 3.

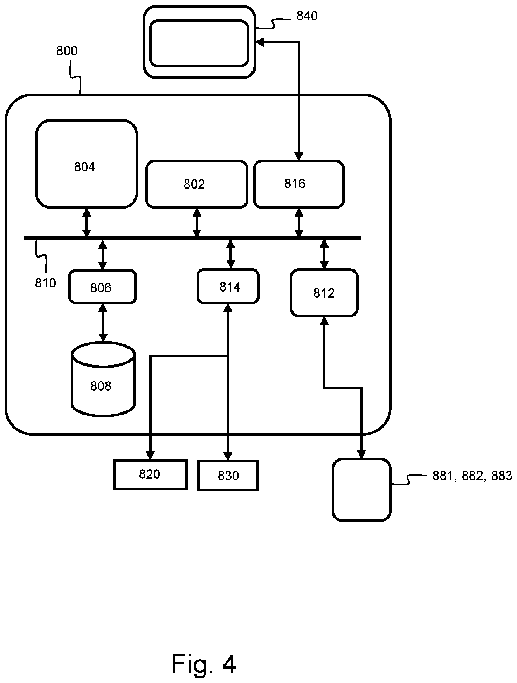

Fig. 4 shows a suitable computing system 800 enabling to implement embodiments of the system. Computing system 800 may in general be formed as a suitable general-purpose computer and comprise a bus 810, a processor 802, a local memory 804, one or more optional input interfaces 814, one or more optional output interfaces 816, a communication interface 812, a storage element interface 806, and one or more storage elements 808. Bus 810 may comprise one or more conductors that permit communication among the components of the computing system 800. Processor 802 may include any type of conventional processor or microprocessor that interprets and executes programming instructions. Local memory 804 may include a random-access memory (RAM) or another type of dynamic storage device that stores information and instructions for execution by processor 802 and/or a read only memory (ROM) or another type of static storage device that stores static information and instructions for use by processor 802. Input interface 814 may comprise one or more conventional mechanisms that permit an operator or user to input information to the computing device 800, such as a keyboard 820, a mouse 830, a pen, voice recognition and/or biometric mechanisms, a camera, etc. Output interface 816 may comprise one or more conventional mechanisms that output information to the operator or user, such as a display 840, etc. Communication interface 812 may comprise any transceiver-like mechanism such as for example one or more Ethernet interfaces that enables computing system 800 to communicate with other devices and/or systems, for example with other computing devices 881, 882, 883. The communication interface 812 of computing system 800 may be connected to such another computing system by means of a local area network (LAN) or a wide area network (WAN) such as for example the internet. Storage element interface 806 may comprise a storage interface such as for example a Serial Advanced Technology Attachment (SATA) interface or a Small Computer System Interface (SCSI) for connecting bus 810 to one or more storage elements 808, such as one or more local disks, for example SATA disk drives, and control the reading and writing of data to and/or from these storage elements 808. Although the storage element(s) 808 above is/are described as a local disk, in general any other suitable computer-readable media such as a removable magnetic disk, optical storage media such as a CD or DVD, -ROM disk, solid state drives, flash memory cards, ... could be used. Computing system 800 could thus correspond to the analyzing means 103 in the embodiment illustrated by Fig. 1.

As used in this application, the term "circuitry" may refer to one or more or all of the following:
(a) hardware-only circuit implementations such as implementations in only analog and/or digital circuitry and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the scope of the claims are therefore intended to be embraced therein.

It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A system (1) for detection and metrology of an allergen skin reaction of an individual, wherein said system (1) comprises:
- testing means (101) configured to host a body part (10) of said individual and to subject a skin surface area (11) of said body part (10) to an allergen skin reaction test;
- illumination means (102) configured to successively illuminate said skin surface area (11) having been subjected to said allergen skin reaction test under one or more predetermined illumination angles (20) with respect to acquisition means (103);
- said acquisition means (103) fixedly positioned with respect to said skin surface area (11), wherein said acquisition means (103) is configured to image, for each of said predetermined illumination angles (20), said illuminated skin surface (21); and
- analysing means (104) configured to determine one or more illumination-angle-dependent variations (202) in light intensity (201) imaged by said acquisition means (103), wherein said illumination-angle-dependent variations (202) are caused by one or more wheals (2) on said skin surface area (11).

2. A system (1) according to claim 1, wherein:
- said skin surface area (11) extends along a longitudinal direction (3);
- said acquisition means are fixedly positioned with respect to said skin surface area (11) along a receiving direction (4) traverse to said longitudinal direction (3);
- said predetermined illumination angles (20) are formed between said receiving direction (4) and a lighting direction (7) of said illumination means (103), wherein said illumination means (103) tangentially illuminates said skin surface area (11) along said lighting direction (7).

3. A system (1) according to any of the preceding claims, wherein said acquisition means (103) are further configured to capture one or more images (30) of said skin surface area (11) for each of said predetermined illumination angles (20), thereby generating a set of images (31) of said skin surface area (11) for said allergen skin reaction test.

4. A system (1) according to claim 3, wherein analysing means (103) are further configured to process said set of images (31) and to determine, from said set of images (31), one or more of the following:
- reflections (203) generated from said illumination by said wheals (2) on said skin surface area (11);
- shadows (204) generated from said illumination by said wheals (2) on said skin surface area (11);
thereby determining said illumination-angle-dependent variations (202) in light intensity (201) imaged by said acquisition means (103).

5. A system (1) according to any of the preceding claims, wherein said analysing means (104) are further configured to determine a location of said wheals (2) on said skin surface area (11) using said illumination-angle-dependent variations (202) in light intensity (201) imaged by said acquisition means (103).

6. A system (1) according to any of the claims 3 to 5, wherein said analysis means (104) are further configured to determine, from said set of images (31), a size and/or a shape of one or more of said wheals (2) on said skin surface area (11).

7. A system (1) according to any of the claims 2 to 6, wherein said acquisition means (103) are positioned along said receiving direction (4) traverse to said longitudinal direction (3) to image said illuminated skin surface (21) under each of said predetermined illumination angles (20).

8. A system (1) according to any of the preceding claims, wherein said acquisition means (103) are positioned at a fixed predetermined receiving distance from said skin surface (11) area for all of said predetermined illumination angles (20).

9. A system (1) according to any of the preceding claims, wherein said illumination means (102) comprise one or more light sources (112), wherein each light source (112) is configured to illuminate said skin surface area (11); and wherein said system (1) further comprises one or more mounting supports (105) configured to hold said one or more light sources (112).

10. A system (1) according to claims 7 and 9, wherein said one or more light sources (112) and/or said mounting supports (105) are further configured to rotate around said body part (10) in a plane (5) comprising said receiving direction (4) and traverse to said longitudinal direction (3) such that said one or more light sources (112) illuminate said skin surface area (11) under said one or more predetermined illumination angles (20).

11. A system (1) according to claims 7 and 9, wherein said light sources (112) and/or said mounting supports (105) are further configured to rotate around said body part (10) in a plane (6) comprising said longitudinal direction (3) of said body part (10) and said receiving direction (4) such that said one or more light sources (112) illuminate said skin surface area (11) under said one or more predetermined illumination angles (20).

12. A system (1) according to any of the preceding claims, wherein said testing means (101) is configured to simultaneously deposit one allergen at a predetermined position on said skin surface area (11) and incise said skin surface area (11) of said individual at each of said predetermined positions.

13. A system (1) according to any of the preceding claims, wherein said testing means (101)further comprise a clamp (111) configured to maintain said body part (10) substantially immobile when imaging said illuminated skin surface (21) for each of said predetermined illumination angles (20).

14. A method for detection and metrology of an allergen skin reaction of an individual, wherein said method comprises the steps of:
- hosting a body part (10) of said individual;
- subjecting a skin surface area (11) of said body part (10) to an allergen skin reaction test;
- successively illuminating said skin surface area (11) having been subjected to said allergen skin reaction test under one or more predetermined illumination angles (20) with respect to a fixed position;
- imaging said illuminated skin surface (21) from said fixed position with respect to said skin surface area (11) and for each of said predetermined illumination angles (20); and
- determining one or more illumination-angle-dependent variations (202) in light intensity (201) being imaged, wherein said illumination-angle-dependent variations (202) are caused by one or more wheals (2) on said skin surface area (11).

15. A method according to claim 14, wherein said method further comprises the steps of:
- capturing one or more images (30) of said skin surface area (11) for each of said predetermined illumination angles (20), thereby generating a set of images (31) of said skin surface area (11) for said allergen skin reaction test;
- determining on said set of images (31) one or more illumination-angle-dependent variations (202) in light intensity (201) being imaged, wherein said illumination-angle-dependent variations (202) are caused by one or more wheals (2) on said skin surface area (11), thereby detecting presence of one or more wheals (2) on said skin surface area (11); and
- determining, from said set of images (31), a location and/or a size and/or a shape of said wheals (2) on said skin surface area (11).
